(19) Europäisches Patentamt
European Patent Office
Office européen des brevets

(11) EP 4 799 997 A1

(12) **EUROPEAN PATENT APPLICATION**
published in accordance with Art. 153(4) EPC

(43) Date of publication:
**02.09.2026 Bulletin 2026/36**

(21) Application number: **24881965.8**

(22) Date of filing: **28.06.2024**

(51) International Patent Classification (IPC):
***C07C 1/213*** (2006.01) ***B01J 29/40*** (2006.01)
***C07B 61/00*** (2006.01) ***C07C 4/06*** (2006.01)
***C07C 11/04*** (2006.01) ***C07C 11/06*** (2006.01)
***C07C 15/04*** (2006.01)

(52) Cooperative Patent Classification (CPC):
**B01J 29/40; C07B 61/00; C07C 1/213; C07C 4/06; C07C 11/04; C07C 11/06; C07C 15/04**

(86) International application number:
**PCT/JP2024/023686**

(87) International publication number:
**WO 2025/088847 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.10.2023 JP 2023181980**

(71) Applicant: **Resonac Corporation**
**Tokyo 105-7325 (JP)**

(72) Inventors:
- **TSUBAKI, Shuntaro**
  **Fukuoka-shi, Fukuoka 819-0395 (JP)**
- **EINAGA, Hisahiro**
  **Fukuoka-shi, Fukuoka 819-0395 (JP)**
- **OTA, Shunsuke**
  **Fukuoka-shi, Fukuoka 819-0395 (JP)**

(74) Representative: **Hoffmann Eitle**
**Patent- und Rechtsanwälte PartmbB**
**Arabellastraße 30**
**81925 München (DE)**

# (54) METHOD FOR PRODUCING COMPOUND, DEVICE FOR PRODUCING COMPOUND, AND ZEOLITE

(57) A method for producing a compound includes performing contact decomposition of an organic compound with zeolite irradiated with microwaves, thereby producing at least one selected from the group consisting of a lower olefin and an aromatic compound.

FIG.1

100
8
4
5a
5
5b
6
6a
6b
G
1
2
3
2
7B
7a
7b
7A
7c
7d

EP 4 799 997 A1

**Description**

TECHNICAL FIELD

The present disclosure relates to a method for producing a compound, a device for producing a compound, and zeolite.

BACKGROUND ART

Basic chemicals, such as ethylene, propylene, benzene, toluene, xylene, and the like, are mainly produced from naphtha derived from petroleum, which is a fossil resource. These basic chemicals are produced by steam-cracking naphtha having 5 carbon atoms to 10 carbon atoms at a reaction temperature of 850°C to 900°C, followed by separation through distillation purification.

In recent years, from the viewpoint of carbon neutrality, studies on substitution to fossil resources have been progressing. One of them is a study on producing basic chemicals using, as raw materials, biomass, such as fats and oils, wax esters, and the like. When fats and oils, and wax esters, are used as raw materials, the basic chemicals are produced by: a method of producing hydrocarbons through hydrocracking and decomposing the produced hydrocarbons in a manner similar to naphtha cracking; or a method of directly pyrolyzing the raw materials.

The method including the production of hydrocarbons is actively studied since this method can be introduced into conventional naphtha cracking and thus can perform the production without significant changes. For example, when fats and oils are used as the raw materials, studies have been conducted on performing hydrodecarboxylation using a nickel-molybdenum catalyst to produce hydrocarbons referred to as bio-naphtha having 16 carbon atoms to 18 carbon atoms, followed by cracking, thereby producing basic chemicals (see, for example, Patent Literatures 1 and 2). Also, as a method for converting wax esters to bio-naphtha, a method for hydrocracking myristyl myristate has been studied, and this method produces hydrocarbons having 14 carbon atoms (see Patent Literature 3).

The method including the production of bio-naphtha has advantages, such as an ability to utilize existing facilities, an ability to produce substantially the same product in quality, and the like. However, the production of bio-naphtha increases the number of steps, and requires use of hydrogen. Thus, this method is not desirable from the viewpoint of carbon neutrality, although it is a transiently necessary technique.

It is very desirable to produce basic chemicals by directly pyrolyzing fats and oils, and wax esters, but currently, a technically sufficient level is not reached. For example, Patent Literature 4 describes a contact decomposition method of waste cooking oil. This study discloses that the decomposition performed at 350°C to 450°C using an FCC catalyst produces linear and branched hydrocarbons having 9 carbon atoms to 24 carbon atoms (olefins: 20 wt% to 40 wt%, and paraffins: 60 wt% to 80 wt%) and aromatic hydrocarbons of 4 wt% to 5 wt%. This method has issues that the decomposition is insufficient, i.e., the number of carbon atoms remains large, and the yield of olefins is low.

Conventional heating is external heating in which the exterior of a furnace is heated by electricity or the like, thereby heating a reaction tube. Whereas, heating using microwaves is internal heating in which a heating target substance, which is a dielectric material, serves as a heating element. When hydrocarbons or the like are reacted using microwaves, a susceptor of carbon or the like that generates heat through absorption of microwaves is used since the hydrocarbons are not heated through absorption of microwaves. In such a method of heating the susceptor by microwaves, selective heating, i.e., heating only a necessary portion, is possible. This is an advantage of microwave heating, i.e., an ability to save input energy without requiring heating the whole furnace as in conventional heating.

A reaction example of such is a reaction for synthesizing benzene from methane at 600°C using a catalyst in which Mo is carried on ZSM-5 of a proton type (see Patent Literature 5). It is reported that, when using a catalyst system in which a catalyst is physically mixed with activated carbon serving as a susceptor, substantially no reaction proceeds in the case of external heating using an electric furnace, but a sufficient reaction proceeds in the case of being heated by irradiation with microwaves of 2.45 GHz. This describes that a reaction can proceed at a low temperature by use of microwaves.

Decomposition of biomass using microwaves is also disclosed (see Patent Literature 6). This study uses a composite material containing a carbon material supported on an inorganic porous framework, and decomposes palm oil in a nitrogen atmosphere at 700 W while intensely generating arc discharge with microwaves of 2.45 GHz. The generated gas is disclosed to be: 13 vol% of methane, 4 vol% of ethane, 49 vol% of ethylene, 16 vol% of propylene, 5 vol% of 1-butene, 8 vol% of 1,3-butadiene, and 5 vol% of the others. In general, the temperature of arc discharge is 1,000°C or higher, which means that the reaction proceeds at a very high temperature.

CITATION LIST

PATENT LITERATURE

Patent Literature 1: PCT Japanese Translation Patent Publication No. 2011-526640
Patent Literature 2: PCT Japanese Translation Patent Publication No. 2018-522086
Patent Literature 3: Japanese Patent No. 5833634
Patent Literature 4: Japanese Patent No. 5234456
Patent Literature 5: International Publication No. WO2012/121366
Patent Literature 6: PCT Japanese Translation Patent Publication No. 2022-506131

SUMMARY OF THE INVENTION

PROBLEM TO BE SOLVED BY THE INVENTION

However, in any of the methods disclosed in the related art documents, no studies have been conducted about, in the method for directly producing useful basic chemicals through pyrolysis of organic compounds, directly heating zeolite with microwaves to enable pyrolysis at a low temperature, and reducing by-product paraffins to increase the yield of olefins having 2 carbon atoms to 3 carbon atoms.

It is an object of the present disclosure to provide a method for producing a compound in which by-product paraffins having 2 carbon atoms to 3 carbon atoms are reduced and the yield of olefin products having 2 carbon atoms to 3 carbon atoms is excellent.

MEANS FOR SOLVING THE PROBLEM

Means for solving the problems are as follows.

<1> A method for producing a compound, the method including:
performing contact decomposition of an organic compound with zeolite irradiated with microwaves, thereby producing at least one selected from the group consisting of a lower olefin and an aromatic compound.
<2> A method for producing a compound, the method including:
performing decomposition of an organic compound with zeolite irradiated with microwaves, thereby producing at least one selected from the group consisting of a lower olefin and an aromatic compound.
<3> The method for producing the compound according to <1> or <2>, wherein the organic compound is at least one selected from the group consisting of a fatty acid, a fatty acid ester, and a hydrocarbon.
<4> The method for producing the compound according to any one of <1> to <3>, wherein the zeolite is at least one selected from the group consisting of an aluminosilicate, a borosilicate, a gallosilicate, and a silicoaluminophosphate.
<5> The method for producing the compound according to any one of <1> to <4>, wherein the zeolite is at least one selected from the group consisting of ZSM-5 and Y.
<6> The method for producing the compound according to any one of <1> to <5>, wherein the zeolite is ZSM-5.
<7> The method for producing the compound according to any one of <1> to <6>, wherein the zeolite contains an alkali metal.
<8> The method for producing the compound according to any one of <1> to <7>, wherein a temperature of the zeolite is 400°C to 600°C.
<9> The method for producing the compound according to any one of <1> to <8>, wherein a frequency of the microwaves is 300 MHz to 300 GHz.
<10> The method for producing the compound according to any one of <1> to <9>, wherein a molecular weight of the organic compound is higher than a molecular weight of the lower olefin and a molecular weight of the aromatic compound.
<11> A device for producing a compound, the device including:

a holder configured to hold zeolite; and
an irradiator configured to irradiate the zeolite with microwaves, wherein
the device is configured to perform, in the holder, contact decomposition of an organic compound with the zeolite irradiated with the microwaves by the irradiator, thereby producing at least one selected from the group consisting of a lower olefin and an aromatic compound.

<12> A device for producing a compound, the device including:

a holder configured to hold zeolite; and
an irradiator configured to irradiate the zeolite with microwaves, wherein
the device is configured to perform, in the holder, decomposition of an organic compound with the zeolite irradiated with the microwaves by the irradiator, thereby producing at least one selected from the group consisting of a lower olefin and an aromatic compound.

<13> Zeolite for use in performing contact decomposition of an organic compound by irradiation with microwaves, thereby producing at least one selected from the group consisting of a lower olefin and an aromatic compound.
<14> Zeolite for use in performing decomposition of an organic compound by irradiation with microwaves, thereby producing at least one selected from the group consisting of a lower olefin and an aromatic compound.
<15> A method for using zeolite, the method including: performing contact decomposition of an organic compound by irradiation with microwaves, thereby producing at least one selected from the group consisting of a lower olefin and an aromatic compound.
<16> A method for using zeolite, the method including: performing decomposition of an organic compound by irradiation with microwaves, thereby producing at least one selected from the group consisting of a lower olefin and an aromatic compound.

ADVANTAGEOUS EFFECTS OF THE INVENTION

According to embodiments of the present disclosure, it is possible to provide a method for producing a compound in which by-product paraffins having 2 carbon atoms to 3 carbon atoms are reduced and the yield of olefin products having 2 carbon atoms to 3 carbon atoms is excellent.

BRIEF DESCRIPTION OF THE DRAWINGS

[FIG. 1] FIG. 1 is a schematic cross-sectional diagram illustrating an example of a device for producing a compound according to an embodiment of the present disclosure.
[FIG. 2] FIG. 2 is a schematic cross-sectional diagram illustrating another example of the device for producing the compound according to the embodiment of the present disclosure.

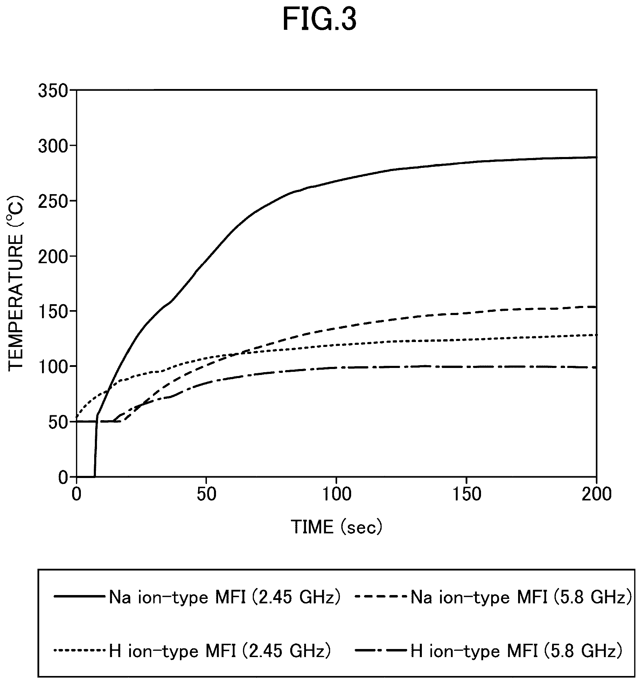

[FIG. 3] FIG. 3 is a graph indicating heating behaviors when Na ion-type MFI and H ion-type MFI are irradiated with an electric field of 2.45 GHz or 5.8 GHz of microwaves, and heated at 500°C for 0 seconds to 200 seconds in Test Example 1. A solid line indicates heating behaviors when the Na ion-type MFI is irradiated with the electric field of 2.45 GHz of microwaves. A dashed line indicates heating behaviors when the Na ion-type MFI is irradiated with the electric field of 5.8 GHz of microwaves. A dotted line indicates heating behaviors when the H ion-type MFI is irradiated with the electric field of 2.45 GHz of microwaves. A dashed-dotted line indicates heating behaviors when the H ion-type MFI is irradiated with the electric field of 5.8 GHz of microwaves. The horizontal axis indicates time (sec) and the vertical axis indicates temperature (°C).

DESCRIPTION OF THE EMBODIMENTS

Hereinafter, embodiments of the present disclosure will be described in detail. The embodiments of the present disclosure are not limited to the following description, and may be modified as appropriate without departing from the gist of the present disclosure. Also, a numerical range indicated by "A to B" in the present disclosure refers to a numerical range including a lower limit "A" and an upper limit "B", unless otherwise specified.

(Method for Producing Compound)

The method for producing the compound according to the embodiment of the present disclosure includes a first embodiment and a second embodiment, which will be described below.

[First Embodiment]

A method for producing a compound according to the first embodiment includes performing contact decomposition of an organic compound with zeolite irradiated with microwaves, thereby producing at least one selected from the group consisting of a lower olefin and an aromatic compound (hereinafter may be referred to as a "microwave irradiation step"); and further includes other steps, if necessary.

According to the method for producing the compound according to the first embodiment, in the method for directly producing useful basic chemicals through pyrolysis of organic compounds, especially fats and oils, hydrocarbons obtained by decarboxylating or hydrodecarboxylating fats and oils, or the like, the zeolite is directly heated with microwaves to enable pyrolysis of the organic compounds at a low temperature and enable efficient production of a lower olefin and an aromatic. Also, by providing a method for producing basic chemicals using biomass, such as fats and oils, and wax esters, it is possible to contribute to suppression of carbon dioxide emission.

<Microwave Irradiation Step>

The microwave irradiation step is a step of performing contact decomposition of an organic compound with zeolite irradiated with microwaves, thereby producing at least one selected from the group consisting of a lower olefin and an aromatic compound.

In the present disclosure, "contact decomposition" and "decompose by contact" means contacting zeolite, which serves as a catalyst, and an organic compound, thereby decomposing the organic compound through a chemical reaction by the effect of the catalyst. In the present disclosure, "contact" means that the zeolite and the organic compound are sufficiently close for decomposing the organic compound to produce at least one selected from the group consisting of a lower olefin and an aromatic compound. In the present disclosure, no particular limitation is imposed on being "close" as long as the effect of the present disclosure is exhibited, i.e., the zeolite exhibits a catalytic effect when decomposing the organic compound to produce at least one selected from the group consisting of a lower olefin and an aromatic compound.

No particular limitation is imposed on conditions of an atmosphere in the microwave irradiation step. The conditions of the atmosphere in the microwave irradiation step can be appropriately selected in accordance with the intended purpose. The atmosphere is preferably an inert gas atmosphere, and more preferably a nitrogen gas atmosphere, an argon gas atmosphere, or the like. Also, the microwave irradiation step can be performed under a water vapor atmosphere like in cracking of naphtha.

The microwave irradiation step, which performs the contact decomposition of the organic compound with the zeolite irradiated with the microwaves, is a reaction between a solid catalyst and a liquid, solid, or gaseous organic compound. Thus, the microwave irradiation step may be a batch reaction. However, when treating a large amount of the organic compound for improvement in production efficiency, the microwave irradiation step is desirably a continuous reaction. In the case of performing the continuous reaction, it is possible to use a method in which the zeolite is disposed in a reaction tube to form a fixed bed, and the organic compound or the like is added to a resulting zeolite layer while allowing an inert gas to flow.

<<Microwaves>>

The microwaves are electromagnetic waves that preferably have a frequency of 300 MHz to 300 GHz. Use of the microwaves can directly transmit energy to the heating target substance, which is a dielectric material. Thus, the heating target substance can behave like a heat source in heating by a conventional electric furnace or the like. Heating by the microwaves is internal heating differing from heating by the electric furnace. In the method for producing the compound according to the first embodiment, the heating target substances of the microwaves are zeolite and organic compounds.

In the microwave irradiation step, the frequency of the microwaves is preferably 300 MHz to 300 GHz as described above, but there is no particular limitation as long as the zeolite can absorb the microwaves and generate heat. Thus, the frequency of the microwaves can be appropriately selected in accordance with the intended purpose, and is more preferably 0.915 GHz to 5.8 GHz and further preferably 2.45 GHz to 5.8 GHz. When the frequency of the microwaves is 300 MHz or more, the zeolite can be heated to an extent sufficient for the contact decomposition of the organic compound. When the frequency of the microwaves is 300 GHz or less, this is preferable from the viewpoint of energy efficiency.

No particular limitation is imposed on a microwave generation source that can be used in the microwave irradiation step, and the microwave generation source can be appropriately selected in accordance with the intended output. Examples of the microwave generation source include diamond SAW (Surface Acoustic Wave), a magnetron, a klystron, a gyrotron, a semiconductor oscillator, and the like. These may be used alone or in combination

Irradiation with the microwaves can be performed without changing at least one of an electric field intensity or a magnetic field intensity from the viewpoint of stabilizing a reaction state, such as reaction activity, selectivity of a product, or the like, and a temperature and temperature distribution of the zeolite. More specifically, for example, the irradiation with the microwaves can be continuously performed without changing the output, frequency, phase, or the like of the microwaves.

No particular limitation is imposed on the output of the microwaves, and the output of the microwaves can be appropriately selected in accordance with the intended temperature in the microwave irradiation step.

<<Zeolite>>

The zeolite is a microporous crystalline aluminosilicate and has a siloxane structure. The zeolite may be a complex in which other metals are complexed. The zeolite absorbs microwaves to generate heat, thereby exhibiting a catalytic effect for pyrolysis of an organic compound.

No particular limitation is imposed on a type of the zeolite, and the type of the zeolite can be appropriately selected in accordance with the intended purpose. The zeolite is preferably at least one selected from the group consisting of an aluminosilicate, a titanosilicate, a borosilicate, a gallosilicate, and a silicoaluminophosphate.

Framework structures of the zeolite are compiled as a database in the International Zeolite Association (hereinafter may be abbreviated as "IZA"), and their IUPAC structure codes (hereinafter may be abbreviated as "structure codes") are defined.

No particular limitation is imposed on the framework structure of the zeolite, and the framework structure of the zeolite can be appropriately selected in accordance with the intended purpose. The framework structure of the zeolite is preferably MFI, MEL, CHA, BEA, FAU, or the like.

Examples of the aluminosilicate include ZSM-5 (Zeolite Socony Mobil-5) in MFI, ZSM-11 in MEL, chabazite in CHA, beta in BEA, Y in FAU, and the like.

Examples of the titanosilicate include TS-1 in MFI.

Examples of the silicoaluminophosphate include SAPO-34 in CHA.

Examples of the borosilicate include Boralite C in MFI.

Examples of the gallosilicate include zeolite in MFI.

These zeolites may be used alone or in combination. Of these, the zeolite is preferably ZSM-5, Y, or beta, and more preferably ZSM-5 or Y.

The crystal system of the zeolite can be analyzed and identified through X-ray diffraction (XRD). Alternatively, the identification of the crystal system of the zeolite can be performed by comparison with the XRD patterns described in the Collection of simulated XRD powder patterns for zeolites, Fifth revised edition (2007), or the XRD patterns described in the Zeolite Framework Types on the website of the Structure Committee of IZA (http://www.iza-struture.org/databases/).

No particular limitation is imposed on a ratio of amounts by mol [$SiO_2/Al_2O_3$] of $SiO_2$ to $Al_2O_3$ in the zeolite, and the ratio of the amounts by mol [$SiO_2/Al_2O_3$] can be appropriately selected in accordance with the intended purpose. The ratio of the amounts by mol [$SiO_2/Al_2O_3$] is preferably 0.1 or greater and 10,000 or less, more preferably 2 or greater and 10,000 or less, further preferably 10 or greater and 5,000 or less, and particularly preferably 20 or greater and 500 or less. When the ratio of the amounts by mol [$SiO_2/Al_2O_3$] is 0.1 or greater and 10,000 or less, the yield of the olefins having 2 carbon atoms to 3 carbon atoms and an O/P ratio of the products having 2 carbon atoms to 3 carbon atoms are favorable.

The ratio of the amounts by mol [$SiO_2/Al_2O_3$] can be calculated, for example, in the following manner. Specifically, zeolite is precisely weighed and completely dissolved using an aqueous solution containing nitric acid and hydrogen fluoride. The resulting solution is made constant in volume to prepare a sample. The prepared sample is measured for the Si and Al contents using an ICP optical emission spectrometer (e.g., PlasmaQuant PQ 9000, available from Analytik Jena AG). The amounts by mol of Si and Al calculated from the Si and Al contents are used to calculate the ratio of the amounts by mol [$SiO_2/Al_2O_3$] according to the following formula:

$$\text{Ratio of the amounts by mol } [SiO_2/Al_2O_3] = 2 \times (\text{the amount by mol of Si})/(\text{the amount by mol of Al})$$

The zeolite may be of a proton type, or may be neutralized with an organic base, an alkali metal, or an alkaline earth metal.

No particular limitation is imposed on a metal contained in the zeolite, and the metal may be appropriately selected in accordance with the intended purpose. Examples of the metal contained in the zeolite include lithium, sodium, potassium, rubidium, cesium, francium, calcium, strontium, barium, radium, aluminum, boron, germanium, phosphorus, chromium, titanium, iron, and the like. Of these, the zeolite preferably contains an alkali metal, more preferably contains sodium, potassium, rubidium, and cesium, further preferably contains sodium, potassium, and cesium, and particularly preferably contains sodium.

Further, if necessary, the zeolite may be used in a state of carrying metals other than the above-described metals. Examples of the other metals include, for example: basic oxides, such as zinc oxide, cerium oxide, lanthanum oxide, and the like; and noble metals, such as platinum, palladium, ruthenium, and the like.

The zeolite may be a commercially available product or may be appropriately synthesized by a publicly known method.

In the microwave irradiation step, the zeolite directly absorbs the microwaves to be heated. No particular limitation is imposed on a temperature of the heated zeolite, and the temperature of the heated zeolite can be appropriately selected in accordance with the intended purpose. The temperature of the heated zeolite is preferably 330°C to 600°C,

more preferably 400°C to 600°C, and further preferably 450°C to 550°C. When the temperature of the heated zeolite is 330°C or higher, it is possible to efficiently obtain a catalytic activity. When the temperature of the heated zeolite is 600°C or lower, the zeolite is not decomposed, and a catalytic activity can be successfully obtained. Also, the temperature of the heated zeolite is a reaction temperature at the time of the contact decomposition of the organic compound in the microwave irradiation step. The temperature of the zeolite can be measured using a publicly known temperature gauge, such as a temperature measuring resistor, a thermocouple, or the like.

No particular limitation is imposed on an amount of the zeolite used relative to the amount of the organic compound added, and the amount of the zeolite used can be appropriately selected in accordance with the intended purpose. In terms of a ratio by mass (total flowing organic compound:zeolite), a ratio of 1,000:1 to 1:100 is preferable, a ratio of 100:1 to 1:50 is more preferable, and a ratio of 50:1 to 1:10 is further preferable. Here, the amount of the organic compound added refers to the mass of the organic compound added as a batch when the microwave irradiation step is performed as a batch reaction. When the microwave irradiation step is performed as a continuous reaction, the amount of the organic compound added refers to the total mass of the added organic compound that flowed.

<<Organic Compound>>

The method for producing the compound according to the first embodiment decomposes the organic compound, thereby producing at least one selected from the group consisting of a lower olefin and an aromatic compound. No particular limitation is imposed on the organic compound, and the organic compound can be appropriately selected in accordance with the intended purpose. The organic compound is preferably fats and oils, or hydrocarbons obtained by decarboxylating or hydrodecarboxylating fats and oils, and more preferably at least one selected from the group consisting of a fatty acid, a fatty acid ester, and a hydrocarbon.

No particular limitation is imposed on the fats and oils as long as a fatty acid is contained. The fats and oils can be appropriately selected in accordance with the intended purpose. Examples of the fatty acid include fatty acids, such as palmitic acid, stearic acid, linoleic acid, $\alpha$-linoleic acid, and the like, and esters thereof. Also, in the method for producing the compound according to the first embodiment, the fats and oils include vegetable oils, such as palm oil, rapeseed oil, sunflower oil, soybean oil, linseed oil, castor oil, and the like, and actually-used waste cooking oil.

No particular limitation is imposed on the hydrocarbon, and the hydrocarbon can be appropriately selected in accordance with the intended purpose. The hydrocarbon is preferably an alkene or a wax ester. The alkene is preferably 1-heptadecene, 1-octadecene, or 1-nonadecene, and more preferably 1-octadecene. The wax ester is a substance in which a long-chain fatty acid having 10 carbon atoms to 12 carbon atoms is ester-bonded to an aliphatic alcohol having 8 or more carbon atoms. Examples of the wax ester include, for example, a wax ester formed mainly of myristyl myristate synthesized from carbon dioxide by *Euglena gracilis*, which is one of microalgae.

No particular limitation is imposed on a molecular weight of the organic compound, and the molecular weight of the organic compound can be appropriately selected in accordance with the intended purpose. For decomposing the organic compound to produce at least one selected from the group consisting of a lower olefin and an aromatic compound, the molecular weight of the organic compound is higher than the molecular weight of the lower olefin and the molecular weight of the aromatic compound.

<Other Steps>

No particular limitation is imposed on other steps, and the other steps can be appropriately selected in accordance with the intended purpose. Examples of the other steps include a recovery step, a separation step, and the like.

<<Recovery Step>>

The recovery step is a step of recovering a liquid substance and a gas, which are products containing a lower olefin and an aromatic compound obtained in the microwave irradiation step. No particular limitation is imposed on the recovery method, and the recovery method can be appropriately selected from publicly known methods in accordance with a type of the obtained product. For example, a gaseous product can be separated through pressurized distillation at normal pressure. Also, a liquid hydrocarbon can be separated through vacuum distillation at normal pressure.

<<Separation Step>>

The separation step is a step of separating useful components alone from the liquid substance and gas recovered in the recovery step, i.e., removing unnecessary components.

The substances produced by the method for producing the compound according to the first embodiment are

hydrogen, methane, carbon monoxide, carbon dioxide, ethylene, ethane, propylene, propane, butene, i-butane, n-butane, butadiene, hydrocarbons having 5 or more carbon atoms, benzene, toluene, m-xylene, p-xylene, o-xylene, ethylbenzene, and other alkyl aromatic compounds.

In the method for producing the compound according to the first embodiment, the term "lower olefin" refers to an olefin having 2 carbon atoms to 3 carbon atoms. The lower olefin is a raw material for polyolefins. The polyolefins are suitably used as raw materials in various fields, such as plastic bags, plastic wrap, straws, medical equipment, home appliance casings, erasers, hoses, tires, tubes, CD cases, food trays, food containers, PET bottles, fibers, and the like.

In the method for producing the compound according to the first embodiment, the term "aromatic compound" refers to benzene, toluene, m-xylene, p-xylene, or o-xylene. Aromatic compounds are raw materials for resins. The resins are suitably used as raw materials in various fields, such as CD cases, food trays, food containers, bottles, fibers, and the like.

Hydrogen, methane, and alkanes may be used as fuels.

Alkanes can be returned to pyrolysis in the method for producing the compound according to the first embodiment.

No particular limitation is imposed on a method for separating the useful components from the unnecessary components in the separation step, and the method for the separation can be appropriately selected from publicly known methods in accordance with the types of the obtained products or the types of the unnecessary components.

According to the method for producing the compound described above, it is possible to obtain a useful component in which the amount of by-product paraffins having 2 carbon atoms to 3 carbon atoms is reduced and the yield of olefin products having 2 carbon atoms to 3 carbon atoms is excellent.

In the method for producing the compound according to the first embodiment, the amount of by-product paraffins having 2 carbon atoms to 3 carbon atoms being reduced and the yield of olefin products having 2 carbon atoms to 3 carbon atoms being excellent can be evaluated by determining a ratio of the total yield (%) of the olefin products having 2 carbon atoms to 3 carbon atoms to the total yield (%) of the paraffin products having 2 carbon atoms to 3 carbon atoms, i.e., a ratio represented by [Total yield (%) of the olefin products having 2 carbon atoms to 3 carbon atoms/Total yield (%) of the paraffin products having 2 carbon atoms to 3 carbon atoms] (hereinafter may be referred to as an "O/P ratio"). In the method for producing the compound according to the first embodiment, the term "paraffin" refers to an aliphatic saturated hydrocarbon having 2 carbon atoms to 3 carbon atoms.

No particular limitation is imposed on the O/P ratio, and the O/P ratio can be appropriately selected in accordance with the intended purpose. The O/P ratio is preferably 6.0 or greater, more preferably 7.0 or greater, further preferably 9.0 or greater, and particularly preferably 10.0 or greater. Since a value of the O/P ratio is preferably greater, no particular limitation is imposed on the upper limit, but the O/P ratio is preferably 50.0 or less and more preferably 20.0 or less.

No particular limitation is imposed on the total yield of the olefin products having 2 carbon atoms to 3 carbon atoms, and the total yield can be appropriately selected in accordance with the intended purpose. The total yield of the olefin products having 2 carbon atoms to 3 carbon atoms is preferably 10% by mass or greater, more preferably 20% by mass or greater, and further preferably 30% by mass or greater, relative to the total mass of the compound. Since a value of the total yield of the olefin products having 2 carbon atoms to 3 carbon atoms is preferably greater, no particular limitation is imposed on the upper limit.

[Second Embodiment]

A method for producing a compound according to the second embodiment includes performing decomposition of an organic compound with zeolite irradiated with microwaves, thereby producing at least one selected from the group consisting of a lower olefin and an aromatic compound (hereinafter may be referred to as a "microwave irradiation step"); and further includes other steps, if necessary. The method for producing the compound according to the second embodiment is the same as the method for producing the compound according to the first embodiment except that the microwave irradiation step is performed as follows.

<Microwave Irradiation Step>

The microwave irradiation step is a step of performing decomposition of an organic compound with zeolite irradiated with microwaves, thereby producing at least one selected from the group consisting of a lower olefin and an aromatic compound. That is, the microwave irradiation step included in the method for producing the compound according to the second embodiment is the same as the microwave irradiation step included in the method for producing the compound according to the first embodiment except that the microwave irradiation step included in the method for producing the compound according to the second embodiment performs decomposition of an organic compound with zeolite irradiated with microwaves.

In the present disclosure, the decomposition of the organic compound with the zeolite irradiated with the

microwaves refers to decomposition of the organic compound in the presence of the zeolite irradiated with the microwaves. Being "in the presence of the zeolite" refers to the zeolite, which serves as a catalyst, being present in a reaction field of the organic compound. In the present disclosure, the term "reaction field" refers to the zeolite and the organic compound being present in the same batch when the microwave irradiation step is performed as a batch reaction. Also, in the present disclosure, the term "reaction field" refers to a zeolite layer in a reaction tube when the microwave irradiation step is performed as a continuous reaction.

(Device for Producing Compound)

The device for producing the compound according to the embodiment of the present disclosure includes a first embodiment and a second embodiment, which will be described below.

[First Embodiment]

A device for producing a compound according to the first embodiment includes a holder configured to hold zeolite and an irradiator configured to irradiate the zeolite with microwaves; and further includes other members, if necessary.

The device for producing the compound according to the first embodiment can successfully perform the method for producing the compound according to the first embodiment. Thus, the holder and the irradiator in the device for producing the compound according to the first embodiment can successfully perform the microwave irradiation step in the method for producing the compound according to the first embodiment.

The device for producing the compound according to the first embodiment is configured to perform, in the holder, contact decomposition of the organic compound with the zeolite irradiated with the microwaves by the irradiator, thereby producing at least one selected from the group consisting of a lower olefin and an aromatic compound.

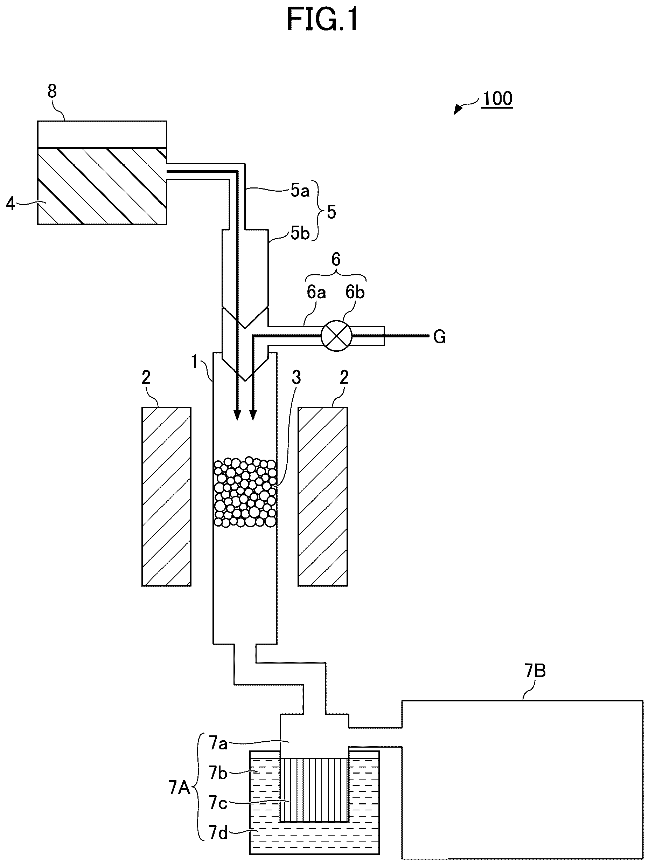

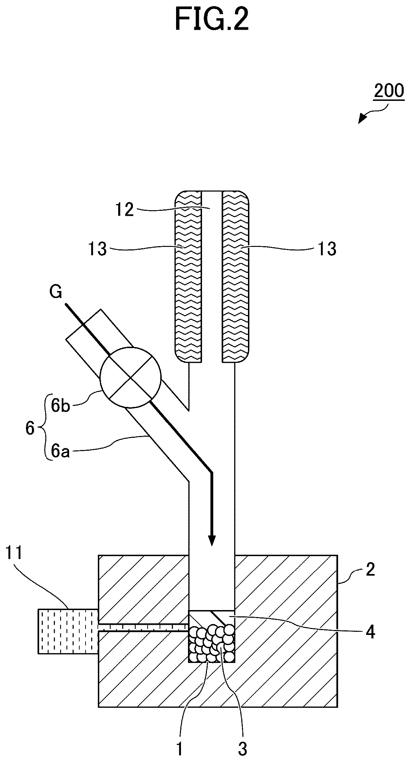

Hereinafter, the device for producing the compound according to the first embodiment (hereinafter may be abbreviated as a "device") will be described with reference to the drawings. FIG. 1 is a schematic cross-sectional diagram illustrating an example of the device for producing the compound according to the first embodiment. FIG. 2 is a schematic cross-sectional diagram illustrating another example of the device for producing the compound according to the first embodiment. The first embodiment described below exemplifies a device that embodies the technical idea of the present disclosure, and should not be construed as limiting the present disclosure to the following. Also, the dimensions, materials, shapes, relative arrangements, and the like of the components described below are not intended to limit the scope of the present disclosure thereto, but are intended to be exemplary unless otherwise specified. In addition, the sizes, positional relationships, and the like of the members illustrated in the drawings may be exaggerated for clarifying the description. Further, for avoiding complicating the drawings more than necessary, the schematic cross-sectional diagram omits illustration of some components. Also, parts indicated by the same signs appearing across the different drawings indicate the same or equivalent parts or members.

<First Mode>

In FIG. 1, a device 100 includes a holder 1 and an irradiator 2. The device 100 of FIG. 1 is a continuous reaction device configured to continuously apply an organic compound 4 to zeolite 3, thereby performing a reaction. This will be described below as the device of the first mode.

<Holder>

The holder 1 is a member configured to hold the zeolite 3. No particular limitation is imposed on the structure, shape, material, and size of the holder 1 as long as the zeolite 3 can be held and the organic compound 4 can be contacted with the zeolite 3. The structure, shape, material, and size of the holder 1 can be appropriately selected in accordance with the intended purpose. An example of the holder 1 is a columnar tube. For retaining the zeolite 3 in the tube, the tube may be plugged with a material capable of allowing an organic compound to pass through, such as glass wool or the like.

<Irradiator>

The irradiator 2 is a member configured to irradiate the zeolite 3 with microwaves. No particular limitation is imposed on the irradiator 2, and a publicly known microwave generator can be used.

<Other Members>

No particular limitation is imposed on other members, and the other members can be appropriately selected in

accordance with the intended purpose. Examples of the other members include an organic compound supply 5, a gas supply 6, a product recovery portion 7, an organic compound reservoir 8, a meter configured to measure the yield of a lower olefin and an aromatic compound, a thermometer configured to measure the temperature of the holder 1, and the like.

<<Organic Compound Supply>>

The organic compound supply 5 is a member configured to supply the organic compound 4 to the holder 1. The organic compound supply 5 includes an organic compound flow portion 5a configured to allow the organic compound 4 to flow, and an introduction portion 5b configured to introduce the organic compound 4 into the holder 1. The organic compound 4 is preferably supplied in a fixed amount and at a fixed rate, and the organic compound supply 5 may include a publicly known pump or the like.

<<Gas Supply>>

The gas supply 6 is a member configured to supply an inert gas G to the holder 1. The gas supply 6 includes a gas flow portion 6a configured to allow the inert gas G to flow, and a pump 6b configured to cause the inert gas G to flow in a fixed amount and for a fixed period.

<Recovery Portion>

The recovery portion 7 is a member configured to recover a product containing at least one selected from the group consisting of the lower olefin and the aromatic compound produced by the device for producing the compound according to the first embodiment. As described above, the product is recovered in the form of liquid or gas. Therefore, the number of the recovery portions 7 may be only one, or two or more. Examples of the two or more recovery portions 7 include a liquid product recovery portion 7A, a gas recovery portion 7B, and the like.

No particular limitation is imposed on the structure, shape, material, and size of the recovery portion 7, and the structure, shape, material, and size of the recovery portion 7 can be appropriately selected in accordance with the intended purpose and a type of the product. The recovery portion 7 is, for example, a publicly known container.

Useful components may be recovered by cooling a liquid product. In this case, the recovery portion 7A for the liquid product may include a trap 7a configured to cool the liquid product, and a cooling portion 7b configured to cool the trap 7a. No particular limitation is imposed on the structure, shape, material, and size of the trap 7a and the cooling portion 7b as long as the trap 7a and the cooling portion 7b can cool the liquid product. The structure, shape, material, and size of the trap 7a and the cooling portion 7b can be appropriately selected in accordance with the intended purpose.

No particular limitation is imposed on the cooling portion 7b as long as the cooling portion 7b can cool the trap 7a. For example, the cooling portion 7b may contain a coolant 7d. The coolant 7d is, for example, ice water.

Also, a solvent 7c capable of separating useful components may be contained in the recovery portion 7A for the liquid product. No particular limitation is imposed on the solvent 7c, and the solvent 7c may be appropriately selected in accordance with types of the useful components to be recovered. The solvent 7c is preferably a nonaqueous solvent. Examples of the nonaqueous solvent include, for example, aromatic organic solvents, such as monochlorobenzene, o-dichlorobenzene, mesitylene, and the like.

The useful components dissolved in the nonaqueous solvent can be successfully separated by further performing distillation at normal pressure.

No particular limitation is imposed on the structure, shape, material, and size of the gas recovery portion 7B, and the structure, shape, material, and size of the gas recovery portion 7B can be appropriately selected in accordance with the intended purpose and types of the products. The gas recovery portion 7B is, for example, a publicly known container.

Also, a solvent or the like capable of separating the useful components may be contained in the gas recovery portion 7B. No particular limitation is imposed on the solvent, and the solvent can be appropriately selected in accordance with types of the useful components to be recovered. Examples of the solvent include ethanol, hexane, dimethylformamide, water, and the like.

The useful components in the gas can be successfully separated by further performing pressurized distillation.

<<Reservoir of Organic Compound>>

The reservoir 8 is a member configured to store an organic compound. No particular limitation is imposed on the structure, shape, material, and size of the reservoir 8 as long as the reservoir 8 can store the organic compound. The structure, shape, material, and size of the reservoir 8 can be appropriately selected in accordance with the intended purpose.

No particular limitation is imposed on the number of the reservoirs 8, and the number of the reservoirs 8 may be

one or more. When the device 100 includes two or more of the reservoirs 8, the two or more reservoirs 8 can be used, for example, to store organic compounds having different compositions or mixtures thereof.

<<Meter>>

The meter is a member configured to measure the yield of the lower olefin and the yield of the aromatic compound in the product containing at least one selected from the group consisting of the lower olefin and the aromatic compound produced by the device for producing the compound according to the first embodiment.

The meter may be provided in the interior of the device 100 or may be connected to the exterior of the device 100.

No particular limitation is imposed on the meter as long as the meter can measure the yield of the lower olefin and the yield of the aromatic compound in the product. The meter may be a publicly known device, e.g., a flame ionization detector (FID), a thermal conductivity detector (TCD), or the like.

No particular limitation is imposed on the structure, shape, material, and size of the meter, and the structure, shape, material, and size of the meter can be appropriately selected in accordance with the intended purpose and types of the products.

<Second Mode>

In FIG. 2, a device 200 includes the holder 1 and the irradiator 2. The device 200 of FIG. 2 is a batch-type reaction device configured to store the zeolite 3 and the organic compound 4 as a batch, thereby performing a reaction. This will be described below as the device of the second mode.

<Holder>

The holder 1 of the device 200 according to the second mode is the same as the device 100 according to the first mode except that the holder 1 of the device 200 is of a batch type. The holder 1 in the device 200 also has the function of the product recovery portion 7 (especially, the trap 7a) of the device 100.

<Irradiator>

The irradiator 2 is the same as that in the device 100 according to the first mode. The irradiator 2 preferably includes the holder 1 in the interior, and more preferably has the function of cooling the holder 1. When the irradiator 2 has the function of cooling the holder 1, performing cooling after completion of a reaction can successfully recover a liquid substance, i.e., a product, in the holder 1. Therefore, the irradiator 2 in the device 200 also has the function of the product recovery portion 7 (especially, the cooling portion 7b) of the device 100.

<Other Members>

No particular limitation is imposed on other members, and the other members can be appropriately selected in accordance with the intended purpose. Examples of the other members include the gas supply 6, the gas recovery portion 7B, a thermometer 11, a gas take-out portion 12, a condenser 13, and a meter configured to measure the yield of the lower olefin and the yield of the aromatic compound. The gas supply 6, the gas recovery portion 7B, and the meter are the same as those in the device 100 according to the first mode.

<<Thermometer>>

The thermometer 11 is configured to measure the temperature of the holder 1. The thermometer may be provided in the interior of the device 200 or may be connected to the exterior of the device 200. The thermometer 11 may be a publicly known thermometer, such as an infrared radiation thermometer or the like.

<<Gas Take-Out Portion>>

The gas take-out portion 12 is a member configured to take out a gas, which is a product. The gas produced in the holder 1 is taken out from the gas take-out portion 12. The gas take-out portion 12 is preferably connected to the gas recovery portion 7B. Also, the gas take-out portion 12 may be connected to the meter, and to the gas recovery portion 7B from the meter. Alternatively, the gas take-out portion 12 may be connected to the gas recovery portion 7B, and to the meter from the gas recovery portion 7B.

<<Condenser>>

The condenser 13 has the function of cooling a gas. The condenser 13 preferably cools the gas take-out portion 12 directly or indirectly. The condenser 13 may be a publicly known condenser.

[Second Embodiment]

A device for producing a compound according to the second embodiment includes a holder configured to hold zeolite and an irradiator configured to irradiate the zeolite with microwaves, and further includes other members if necessary.

The device for producing the compound according to the second embodiment can successfully perform the method for producing the compound according to the second embodiment. Thus, the holder and the irradiator in the device for producing the compound according to the second embodiment can successfully perform the microwave irradiation step in the method for producing the compound according to the second embodiment.

The device for producing the compound according to the second embodiment is configured to perform, in the holder, decomposition of the organic compound with the zeolite irradiated with the microwaves by the irradiator, thereby producing at least one selected from the group consisting of a lower olefin and an aromatic compound. That is, the device for producing the compound according to the second embodiment is the same as the device for producing the compound according to the first embodiment, except that the decomposition is performed in the holder with the zeolite irradiated with the microwaves by the irradiator in the second embodiment while the contact decomposition is performed in the holder with the zeolite irradiated with the microwaves by the irradiator in the first embodiment.

(Zeolite and Method for Using Zeolite)

Zeolite according to an embodiment of the present disclosure includes a first embodiment and a second embodiment, which will be described below.

[First Embodiment]

Zeolite according to the first embodiment is for use in performing contact decomposition of an organic compound by irradiation with microwaves, thereby producing at least one selected from the group consisting of a lower olefin and an aromatic compound. Therefore, a method for using zeolite including performing contact decomposition of an organic compound by irradiation with microwaves, thereby producing at least one selected from the group consisting of a lower olefin and an aromatic compound, is also within the scope of the present disclosure.

Microwaves, organic compounds, lower olefins, and aromatic compounds are as described in the above section (Method for Producing Compound), and therefore, detailed description thereof will be omitted.

[Second Embodiment]

Zeolite according to the second embodiment is for use in performing decomposition of an organic compound by irradiation with microwaves, thereby producing at least one selected from the group consisting of a lower olefin and an aromatic compound. Therefore, a method for using zeolite including performing decomposition of an organic compound by irradiation with microwaves, thereby producing at least one selected from the group consisting of a lower olefin and an aromatic compound, is also within the scope of the present disclosure.

The zeolite and the method for using the zeolite according to the second embodiment are the same as the zeolite and the method for using the zeolite according to the first embodiment, except that the zeolite is used for performing the decomposition of the organic compound by irradiation with the microwaves in the second embodiment while the zeolite is used for performing the contact decomposition of the organic compound by irradiation with the microwaves in the first embodiment.

EXAMPLES

The embodiments of the present disclosure will be described below in detail by way of Examples, Comparative Examples, and Test Examples, but the embodiments of the present disclosure are not limited to these Examples, Comparative Examples, and Test Examples.

(Example 1)

Glass wool was packed into a cylindrical quartz tube having an inner diameter of 8 mm (hereinafter may be referred to as a "reaction tube"), and 100 mg of Na ion-type MFI (Na-ZSM-5, Silton™ Mizuka Sieves EX-122, a ratio of amounts by mol [$SiO_2/Al_2O_3$]=30, obtained from MIZUSAWA INDUSTRIAL CHEMICALS, LTD.) was packed as catalyst zeolite onto the glass wool. This reaction tube was irradiated with an electric field of 5.8 GHz of microwaves using a microwave generator (MR-5G-50, obtained from Ryowa Electronics Co., Ltd.) to increase the temperature to 500°C. While a nitrogen gas was allowed to flow from an upper portion of the reaction tube at 10 mL/min, methyl oleate (boiling point: 218.5°C, melting point: 19.9°C, molecular weight: 296.49, and density at 25°C: 0.874 g/cm$^3$) serving as a reaction substrate was dropped onto an upper portion of the zeolite layer at 30 μL/min, thereby performing a reaction (the amount of methyl oleate added for 60 minutes was 1.56 g). The product was divided into a liquid substance and a gas at room temperature (20°C±5°C), and quantitatively analyzed for the components shown in Table 1 below. The liquid substance of the product was absorbed into ethanol disposed at a lower portion of the reaction tube for 15 minutes. Then, gas chromatography analysis using a flame ionization detector (FID) was performed using d-limonene (obtained from Sigma-Aldrich Japan, 97%) as an internal standard under the following analysis conditions.

[Analysis Conditions]

Device: Gas chromatograph (Model: GC-14B, obtained from Shimadzu Corporation)
Column: CP-Sil5 CB (inner diameter: 0.25 mm, length: 60 m, film thickness: 0.25 μm, obtained from Agilent Co.)
Detector: FID
Carrier gas: He
Injection volume: 1 μL
Heating program: heating from 50°C to 120°C at 5 °C/min → retaining at 120°C for 5 minutes → heating to 150°C at 5 °C/min → retaining at 150°C for 1 minute
Detector temperature: 260°C

The gas of the product was in-line analyzed through micro GC using a thermal conductivity detector (TCD) as a detector under the following analysis conditions.

[Analysis Conditions]

Device: 490 Micro GC (obtained from Agilent Technologies)
Column: CH1: MS5A column
CH2: Porapak Q
Detector: TCD
Carrier gas: CH1: Ar
CH2: He
Column temperature: CH1: 100°C
CH2: 80°C

The results of the quantitative analysis in Example 1 are shown in Table 1 below. In Table 1, the results of the quantitative analysis are shown as the total carbon yield for every 15 minutes: 0 minutes or greater and 15 minutes or less; greater than 15 minutes and equal to or less than 30 minutes; greater than 30 minutes and equal to or less than 45 minutes; and greater than 45 minutes and equal to or less than 60 minutes. The carbon yield and the O/P ratio of the products having 2 carbon atoms to 3 carbon atoms were defined as follows:

Carbon yield = (Amount by mol of carbon in the product)/(Amount by mol of carbon of the substance charged into the reaction tube at each time);

and

O/P ratio of the products having 2 carbon atoms to 3 carbon atoms = (Amount by mol of carbon of ethylene in the product + Amount by mol of carbon of propylene in the product)/(Amount by mol of carbon of ethane in the product + Amount by mol of carbon of propane in the product).

[Table 1]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 2.12 | 3.53 | 1.85 | 1.36 |
| | Methane | 0.00 | 0.00 | 0.00 | 0.02 |
| | Ethylene | 21.93 | 43.57 | 24.02 | 17.11 |
| | Ethane | 2.13 | 4.13 | 2.44 | 2.63 |
| | Propylene | 6.54 | 10.73 | 6.52 | 3.80 |
| | Propane | 0.37 | 0.69 | 0.39 | 0.28 |
| | i-Butane | 0.00 | 0.00 | 0.00 | 0.05 |
| | n-Butane | 0.35 | 0.39 | 0.21 | 0.08 |
| | Benzene | 2.90 | 5.45 | 5.63 | 3.48 |
| | Toluene | 0.73 | 1.31 | 1.19 | 1.82 |
| | m+p-Xylene | 0.00 | 0.09 | 0.15 | 0.54 |
| | o-Xylene | 0.07 | 0.17 | 0.18 | 0.88 |
| Total yield [% by mass] | | 37.14 | 70.06 | 42.58 | 32.05 |
| O/P ratio of products having 2 carbon atoms and 3 carbon atoms | | 11.39 | 11.27 | 10.79 | 7.19 |
| Yield [% by mass] of olefins having 2 carbon atoms and 3 carbon atoms | | 28.47 | 54.30 | 30.54 | 20.91 |
| Yield [% by mass] of useful aromatics | | 3.70 | 7.02 | 7.15 | 6.72 |
| Yield [% by mass] of paraffins having 2 carbon atoms and 3 carbon atoms | | 2.50 | 4.82 | 2.83 | 2.91 |

(Comparative Example 1)

A reaction was performed in the same manner as in Example 1 except that, unlike in Example 1, heating to 500°C was performed in an electric furnace (ceramic electric tubular furnace, obtained from Asahi RIKA Co., Ltd.) instead of increasing the temperature to 500°C by irradiation with the electric field of 5.8 GHz of the microwaves using the microwave generator. Then, quantitative analysis of the components shown in Table 2 below was performed in the same manner as in Example 1. The results of the quantitative analysis in Comparative Example 1 are shown in Table 2.

[Table 2]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 1.36 | 1.18 | 1.10 | 0.74 |
| | Methane | 0.04 | 0.02 | 0.02 | 0.00 |
| | Ethylene | 4.67 | 3.73 | 3.81 | 2.31 |
| | Ethane | 1.30 | 1.05 | 1.10 | 0.67 |
| | Propylene | 6.81 | 4.36 | 3.50 | 2.44 |
| | Propane | 0.28 | 0.21 | 0.20 | 0.15 |
| | i-Butane | 0.02 | 0.02 | 0.02 | 0.00 |
| | n-Butane | 0.59 | 0.39 | 0.42 | 0.26 |
| | Benzene | 1.15 | 1.23 | 1.29 | 1.06 |
| | Toluene | 1.68 | 1.89 | 2.09 | 2.20 |
| | m+p-Xylene | 1.56 | 2.00 | 2.33 | 1.73 |
| | o-Xylene | 0.09 | 0.24 | 0.35 | 0.46 |
| Total yield [% by mass] | | 19.55 | 16.32 | 16.23 | 12.02 |
| O/P ratio of products having 2 carbon atoms and 3 carbon atoms | | 7.27 | 6.42 | 5.62 | 5.79 |
| Yield [% by mass] of olefins having 2 carbon atoms and 3 carbon atoms | | 11.48 | 8.09 | 7.31 | 4.75 |
| Yield [% by mass] of useful aromatics | | 4.48 | 5.36 | 6.06 | 5.45 |
| Yield [% by mass] of paraffins having 2 carbon atoms and 3 carbon atoms | | 1.58 | 1.26 | 1.30 | 0.82 |

(Comparative Example 2)

A reaction was performed in the same manner as in Comparative Example 1 except that, unlike in Comparative Example 1, the heating to 500°C in the electric furnace was changed to heating to 600°C in the electric furnace. Then, quantitative analysis of the components shown in Table 3 below was performed in the same manner as in Comparative Example 1. The results of the quantitative analysis in Comparative Example 2 are shown in Table 3.

[Table 3]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 0.82 | 0.94 | 1.13 | 1.09 |
| | Methane | 0.31 | 0.13 | 0.08 | 0.07 |
| | Ethylene | 5.49 | 6.07 | 6.33 | 6.54 |
| | Ethane | 1.92 | 2.09 | 2.28 | 2.30 |
| | Propylene | 1.84 | 2.20 | 2.96 | 2.48 |
| | Propane | 0.16 | 0.19 | 0.26 | 0.23 |
| | i-Butane | 0.00 | 0.00 | 0.00 | 0.00 |
| | n-Butane | 0.07 | 0.10 | 0.19 | 0.12 |
| | Benzene | 2.75 | 3.61 | 4.06 | 4.02 |
| | Toluene | 1.46 | 2.24 | 2.81 | 2.68 |
| | m+p-Xylene | 0.32 | 0.51 | 0.69 | 0.63 |
| | o-Xylene | 0.15 | 0.32 | 0.43 | 0.39 |
| Total yield [% by mass] | | 15.29 | 18.40 | 21.22 | 20.55 |
| O/P ratio of products having 2 carbon atoms and 3 carbon atoms | | 3.52 | 3.63 | 3.66 | 3.57 |
| Yield [% by mass] of olefins having 2 carbon atoms and 3 carbon atoms | | 7.33 | 8.27 | 9.29 | 9.02 |
| Yield [% by mass] of useful aromatics | | 4.68 | 6.68 | 7.99 | 7.72 |
| Yield [% by msss] of paraffins having 2 carbon atoms and 3 carbon atoms | | 2.08 | 2.28 | 2.54 | 2.53 |

When comparing Example 1 with Comparative Examples 1 and 2, the activity in Example 1, in which the heating was preformed with microwaves, was found to be high since useful products, such as ethylene, propylene, benzene, toluene, m-xylene, p-xylene, o-xylene, and the like, were more than in Comparative Examples 1 and 2. Also, the O/P ratio of the products having 2 carbon atoms to 3 carbon atoms in Example 1, in which the decomposition was performed by the heating with the microwaves, was much higher than that in Comparative Examples 1 and 2, in which the decomposition was performed by the heating in the electric furnace. Although a reason for this is not clearly understood, one possible reason is because of selective and local heating of the zeolite with the microwaves.

(Example 2)

A reaction was performed in the same manner as in Example 1 except that, unlike in Example 1, 30 μL/min of methyl oleate serving as the reaction substrate in Example 1 was changed to 30 μL/min of 1-octadecene (boiling point: 179°C, melting point: 16°C, molecular weight: 252.48, and density at 25°C: 0.789 g/cm$^3$) (the amount of 1-octadecene added for 60 minutes was 1.42 g). Then, quantitative analysis of the components shown in Table 4 below was performed in the same manner as in Example 1. The results of the quantitative analysis in Example 2 are shown in Table 4. Note that 1-octadecene is a compound decarboxylated from methyl oleate, and used as a model material of a decarboxylated hydrocarbon.

[Table 4]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 0.01 | 0.01 | 0.01 | 0.01 |
| | Methane | 1.31 | 1.27 | 3.59 | 2.55 |
| | Ethylene | 8.01 | 8.46 | 8.80 | 8.46 |
| | Ethane | 1.18 | 1.11 | 1.34 | 1.43 |
| | Propylene | 5.42 | 3.72 | 2.62 | 2.53 |
| | Propane | 0.22 | 0.17 | 0.14 | 0.14 |
| | i-Butane | 0.02 | 0.01 | 0.00 | 0.00 |
| | n-Butane | 0.53 | 0.27 | 0.13 | 0.13 |
| | Benzene | 0.98 | 0.79 | 1.45 | 1.80 |
| | Toluene | 0.31 | 0.29 | 0.81 | 1.05 |
| | m+p-Xylene | 0.00 | 0.00 | 0.14 | 0.17 |
| | o-Xylene | 0.01 | 0.01 | 0.08 | 0.23 |
| Total yield [% by mass] | | 18.00 | 16.11 | 19.11 | 18.50 |
| O/P ratio of products having 2 carbon atoms and 3 carbon atoms | | 9.59 | 9.52 | 7.72 | 7.00 |
| Yield [% by mass] of olefins having 2 carbon atoms and 3 carbon atoms | | 13.43 | 12.18 | 11.42 | 10.99 |
| Yield [% by mass] of useful aromatics | | 1.30 | 1.09 | 2.48 | 3.25 |
| Yield [% by mass] of paraffins having 2 carbon atoms and 3 carbon atoms | | 1.40 | 1.28 | 1.48 | 1.57 |

(Comparative Example 3)

A reaction was performed in the same manner as in Example 2 except that, unlike in Example 2, heating to 500°C was performed in an electric furnace (ceramic electric tubular furnace, obtained from Asahi RIKA Co., Ltd.) instead of increasing the temperature to 500°C by irradiation with the electric field of 5.8 GHz of the microwaves using the microwave generator. Then, quantitative analysis of the components shown in Table 5 below was performed in the same manner as in Example 2. The results of the quantitative analysis in Comparative Example 3 are shown in Table 5.

[Table 5]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 0.00 | 0.00 | 0.00 | 0.00 |
| | Methane | 0.14 | 0.02 | 0.02 | 0.00 |
| | Ethylene | 1.60 | 0.89 | 1.48 | 0.97 |
| | Ethane | 0.71 | 0.34 | 0.57 | 0.35 |
| | Propylene | 2.66 | 1.09 | 1.66 | 0.97 |
| | Propane | 0.14 | 0.06 | 0.09 | 0.06 |
| | i-Butane | 0.00 | 0.00 | 0.00 | 0.00 |
| | n-Butane | 0.31 | 0.13 | 0.18 | 0.13 |
| | Benzene | 0.31 | 0.38 | 0.46 | 0.98 |
| | Toluene | 1.61 | 0.94 | 1.20 | 0.84 |
| | m+p-Xylene | 0.31 | 0.87 | 1.13 | 0.49 |
| | o-Xylene | 0.82 | 1.19 | 0.55 | 0.69 |
| Total yield [% by mass] | | 8.61 | 5.91 | 7.34 | 5.48 |
| O/P ratio of products having 2 carbon atoms and 3 carbon atoms | | 5.01 | 4.95 | 4.76 | 4.73 |
| Yield [% by mass] of olefins having 2 carbon atoms and 3 carbon atoms | | 4.26 | 1.98 | 3.14 | 1.94 |
| Yield [% by mass] of useful aromatics | | 3.05 | 3.38 | 3.34 | 3.00 |
| Yield [% by mass] of paraffins having 2 carbon atoms and 3 carbon atoms | | 0.85 | 0.40 | 0.66 | 0.41 |

(Comparative Example 4)

A reaction was performed in the same manner as in Comparative Example 3 except that, unlike in Comparative Example 3, the heating to 500°C in the electric furnace was changed to heating to 600°C in the electric furnace. Then, quantitative analysis of the components shown in Table 6 below was performed in the same manner as in Comparative Example 3. The results of the quantitative analysis in Comparative Example 4 are shown in Table 6.

[Table 6]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 0.00 | 0.00 | 0.00 | 0.00 |
| | Methane | 1.00 | 0.21 | 0.06 | 0.05 |
| | Ethylene | 7.50 | 6.48 | 5.51 | 5.69 |
| | Ethane | 2.59 | 2.28 | 1.91 | 1.91 |
| | Propylene | 3.31 | 3.20 | 2.86 | 2.58 |
| | Propane | 0.26 | 0.25 | 0.22 | 0.20 |
| | i-Butane | 0.00 | 0.00 | 0.01 | 0.00 |
| | n-Butane | 0.13 | 0.15 | 0.14 | 0.14 |
| | Benzene | 3.46 | 3.51 | 3.50 | 3.42 |
| | Toluene | 1.58 | 1.79 | 1.71 | 1.65 |
| | m+p-Xylene | 0.28 | 0.36 | 0.33 | 0.31 |
| | o-Xylene | 0.23 | 0.28 | 0.26 | 0.26 |
| Total yield [% by mass] | | 20.34 | 18.51 | 16.51 | 16.21 |
| O/P ratio of products having 2 carbon atoms and 3 carbon atoms | | 3.79 | 3.83 | 3.93 | 3.92 |
| Yield [% by mass] of olefins having 2 carbon atoms and 3 carbon atoms | | 10.81 | 9.68 | 8.37 | 8.27 |
| Yield [% by mass] of useful aromatics | | 5.55 | 5.94 | 5.80 | 5.64 |
| Yield [% by mass] of paraffins having 2 carbon atoms and 3 carbon atoms | | 2.85 | 2.53 | 2.13 | 2.11 |

When comparing Example 2 with Comparative Examples 3 and 4, the activity in Example 2, in which the heating was preformed with microwaves, was found to be high since there were more useful products, such as ethylene, propylene, benzene, toluene, m-xylene, p-xylene, o-xylene, and the like, than in Comparative Examples 3 and 4. Also, the results obtained in the heating at 500°C with microwaves were substantially the same as the results obtained in the heating at 600°C in the electric furnace. Further, the O/P ratio of the products having 2 carbon atoms to 3 carbon atoms in Example 2, in which the decomposition was performed by the heating with the microwaves, was much higher than that in Comparative Examples 3 and 4, in which the decomposition was performed by the heating in the electric furnace. These results have the same tendency as seen in the results of the comparison of Example 1 with Comparative Examples 1 and 2.

(Example 3)

A reaction was performed in the same manner as in Example 1 except that, unlike in Example 1, the temperature was increased to 500°C by irradiation with an electric field of 2.45 GHz of microwaves using a microwave generator (MR-2G-100, obtained from Ryowa Electronics Co., Ltd.) instead of increasing the temperature to 500°C by irradiation with the electric field of 5.8 GHz of the microwaves using the microwave generator (MR-5G-50, obtained from Ryowa Electronics Co., Ltd.). Then, quantitative analysis was performed in the same manner as in Example 1. The results of the quantitative analysis in Example 3 are shown in Table 7 below.

[Table 7]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 1.48 | 0.98 | 0.88 | 1.90 |
| | Methane | 1.46 | 0.74 | 0.54 | 1.07 |
| | Ethylene | 22.34 | 15.28 | 13.89 | 29.16 |
| | Ethane | 2.09 | 1.64 | 1.59 | 3.51 |
| | Propylene | 7.25 | 4.87 | 4.21 | 8.75 |
| | Propane | 0.56 | 0.40 | 0.36 | 0.74 |
| | i-Butane | 0.00 | 0.00 | 0.00 | 0.00 |
| | n-Butane | 0.36 | 0.12 | 0.08 | 0.17 |
| | Benzene | 3.18 | 3.10 | 2.38 | 3.15 |
| | Toluene | 0.67 | 0.82 | 0.67 | 1.23 |
| | m+p-Xylene | 0.00 | 0.09 | 0.00 | 0.22 |
| | o-Xylene | 0.10 | 0.06 | 0.05 | 0.28 |
| Total yield [% by mass] | | 39.49 | 28.09 | 24.64 | 50.18 |
| O/P ratio of products having 2 carbon atoms and 3 carbon atoms | | 11.18 | 9.88 | 9.28 | 8.92 |
| Yield [% by mass] of olefins having 2 carbon atoms and 3 carbon atoms | | 29.59 | 20.15 | 18.10 | 37.91 |
| Yield [% by mass] of useful aromatics | | 3.95 | 4.06 | 3.10 | 4.87 |
| Yield [% by mass] of paraffins having 2 carbon atoms and 3 carbon atoms | | 2.65 | 2.04 | 1.95 | 4.25 |

(Example 4)

A reaction was performed in the same manner as in Example 3 except that, unlike in Example 3, the Na ion-type MFI serving as catalyst zeolite was changed to Na ion-type FAU (Y type (JRC-Z-Y5.5), a ratio of amounts by mol [$SiO_2/Al_2O_3$]=5.6, obtained from Tosoh Corporation). Then, quantitative analysis of the components shown in Table 8 below was performed in the same manner as in Example 3. The results of the quantitative analysis in Example 4 are shown in Table 8.

[Table 8]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 0.69 | 0.75 | 0.67 | 0.84 |
| | Methane | 0.00 | 0.00 | 0.00 | 0.00 |
| | Ethylene | 12.94 | 12.53 | 10.74 | 13.31 |
| | Ethane | 1.74 | 1.84 | 1.91 | 2.22 |
| | Propylene | 4.31 | 3.35 | 3.78 | 3.91 |
| | Propane | 0.36 | 0.30 | 0.38 | 0.37 |
| | i-Butane | 0.00 | 0.00 | 0.00 | 0.00 |
| | n-Butane | 0.16 | 0.05 | 0.09 | 0.11 |
| | Benzene | 2.38 | 1.17 | 2.12 | 1.43 |
| | Toluene | 0.66 | 0.26 | 0.95 | 0.53 |
| | m+p-Xylene | 0.21 | 0.00 | 0.20 | 0.07 |
| | o-Xylene | 0.14 | 0.00 | 0.23 | 0.17 |
| Total yield [% by mass] | | 23.59 | 20.25 | 21.07 | 22.96 |
| O/P ratio of products having 2 carbon atoms and 3 carbon atoms | | 8.21 | 7.42 | 6.34 | 6.65 |
| Yield [% by mass] of olefins having 2 carbon atoms and 3 carbon atoms | | 17.25 | 15.88 | 14.52 | 17.22 |
| Yield [% by mass] of useful aromatics | | 3.39 | 1.43 | 3.50 | 2.20 |
| Yield [% by mass] of paraffins having 2 carbon atoms and 3 carbon atoms | | 2.10 | 2.14 | 2.29 | 2.59 |

(Comparative Example 5)

A reaction was performed in the same manner as in Example 4 except that, unlike in Example 4, heating to 500°C was performed in an electric furnace (ceramic electric tubular furnace, obtained from Asahi RIKA Co., Ltd.) instead of increasing the temperature to 500°C by irradiation with the electric field of 2.45 GHz of the microwaves using the microwave generator. Then, quantitative analysis of the components shown in Table 9 below was performed in the same manner as in Example 4. The results of the quantitative analysis in Comparative Example 5 are shown in Table 9.

[Table 9]

| | | Reaction time | | | |
|---|---|---|---|---|---|
| | | 0 min or greater and 15 min or less | Greater than 15 min and equal to or less than 30 min | Greater than 30 min and equal to or less than 45 min | Greater than 45 min and equal to or less than 60 min |
| Yield [% by mass] | $CO_2$ | 0.00 | 0.00 | 0.02 | 0.02 |
| | Methane | 0.00 | 0.00 | 0.00 | 0.00 |
| | Ethylene | 0.00 | 0.02 | 0.12 | 0.19 |
| | Ethane | 0.01 | 0.01 | 0.11 | 0.14 |
| | Propylene | 0.00 | 0.01 | 0.04 | 0.05 |
| | Propane | 0.00 | 0.01 | 0.04 | 0.04 |
| | i-Butane | 0.00 | 0.00 | 0.00 | 0.00 |
| | n-Butane | 0.00 | 0.00 | 0.00 | 0.00 |
| | Benzene | 0.00 | 0.23 | 0.13 | 0.13 |
| | Toluene | 0.27 | 0.68 | 0.31 | 0.14 |
| | m+p-Xylene | 0.09 | 0.41 | 0.17 | 0.05 |
| | o-Xylene | 0.00 | 0.11 | 0.04 | 0.07 |
| Total yield [% by mass] | | 0.37 | 1.48 | 0.98 | 0.83 |
| O/P ratio of products having 2 carbon atoms and 3 carbon atoms | | 0.00 | 1.50 | 1.07 | 1.33 |
| Yield [% by mass] of olefins having 2 carbon atoms and 3 carbon atoms | | 0.00 | 0.03 | 0.16 | 0.24 |
| Yield [% by mass] of useful aromatics | | 0.36 | 1.43 | 0.65 | 0.39 |
| Yield [% by mass] of paraffins having 2 carbon atoms and 3 carbon atoms | | 0.01 | 0.02 | 0.15 | 0.18 |

When comparing Example 4 with Comparative Example 5, the activity in Example 4, in which the heating was preformed with microwaves, was found to be high since there were more useful products, such as ethylene, propylene, benzene, toluene, m-xylene, p-xylene, o-xylene, and the like, than in Comparative Example 5. Also, the O/P ratio of the products (i.e., olefins) having 2 carbon atoms to 3 carbon atoms in Example 4, in which the decomposition was performed by the heating with the microwaves, was much higher than that in Comparative Example 5, in which the decomposition was performed by the heating in the electric furnace. These results have the same tendency as seen in the results of the comparison of Example 1 with Comparative Examples 1 and 2.

(Example 5)

A reaction was performed in the same manner as in Example 4 except that, unlike in Example 4, the Na ion-type FAU serving as catalyst zeolite was changed to H ion-type FAU (Y type (JRC-Z-HY5.5), a ratio of amounts by mol [$SiO_2/Al_2O_3$]=5.6, obtained from Tosoh Corporation). Then, quantitative analysis of the components shown in Table 10 below was performed in the same manner as in Example 4. The results of the quantitative analysis in Example 5 are shown in Table 10. Note that a period for quantification was up to 30 minutes, and in Table 10, the results of the quantitative analysis are shown as the total carbon yields in the range of 0 minutes or greater and 15 minutes or less and in the range of greater than 15 minutes and equal to or less than 30 minutes.

[Table 10]

| | | Reaction time | |
|---|---|---|---|
| | | 0 min or greater and 15 min or less | Greater than 15 min and equal to or less than 30 min |
| Yield [% by mass] | $CO_2$ | 0.30 | 0.16 |
| | Methane | 0.00 | 0.00 |
| | Ethylene | 4.82 | 2.88 |
| | Ethane | 0.65 | 0.31 |
| | Propylene | 2.04 | 0.52 |
| | Propane | 0.18 | 0.06 |
| | i-Butane | 0.00 | 0.00 |
| | n-Butane | 0.15 | 0.01 |
| | Benzene | 1.69 | 0.58 |
| | Toluene | 0.51 | 0.26 |
| | m+p-Xylene | 0.08 | 0.00 |
| | o-Xylene | 1.13 | 0.10 |
| Total yield [% by mass] | | 11.55 | 4.88 |
| O/P ratio of products having 2 carbon atoms and 3 carbon atoms | | 8.27 | 9.19 |
| Yield [% by mass] of olefins having 2 carbon atoms and 3 carbon atoms | | 6.86 | 3.40 |
| Yield [% by mass] of useful aromatics | | 3.41 | 0.94 |
| Yield [% by mass] of paraffins having 2 carbon atoms and 3 carbon atoms | | 0.83 | 0.37 |

When comparing Example 4 with Example 5, the O/P ratios of the products having 2 carbon atoms to 3 carbon atoms were both good values. The result of the total yield was better in Example 4, in which the Na ion-type FAU was used, than in Example 5, in which the H ion-type FAU was used. This indicates that the decomposition with microwaves is more efficiently performed by the Na ion-type FAU than the H ion-type FAU.

(Test Example 1)

The following test samples were each irradiated with an electric field of 2.45 GHz of microwaves using a microwave generator (MR-2G-100, obtained from Ryowa Electronics Co., Ltd.) and an electric field of 5.8 GHz of microwaves using a microwave generator (MR-5G-50, obtained from Ryowa Electronics Co., Ltd.) and heated at 500°C for 0 seconds to 200 seconds. Then, heating behaviors were analyzed by an infrared radiation thermometer (Model: TMHX-STM0050-0070E003-0445, obtained from Japan Sensor Co., Ltd.). The results are shown in FIG. 3.

<Test Samples>

(1) Na ion-type MFI (Na-ZSM-5, Silton™ Mizuka Sieves EX-122, a ratio of amounts by mol [$SiO_2/Al_2O_3$]=30, obtained from MIZUSAWA INDUSTRIAL CHEMICALS, LTD.)
(2) H ion-type MFI (H-ZSM-5, a ratio of amounts by mol [$SiO_2/Al_2O_3$]=30, obtained from MIZUSAWA INDUSTRIAL CHEMICALS, LTD.)

When comparing test sample (1) with test sample (2), the Na ion-type MFI was found to be more readily heated than the H ion-type MFI was. Also, the Na ion-type MFI was found to be more readily heated at 2.45 GHz than at 5.8 GHz.

(Production Example 1)

500 mg of H ion-type MFI (HSZ (registered trademark)-800, grade: 840HOD1A, obtained from Tosoh Corporation) was suspended in 150 mL of a 0.2 mol/L aqueous potassium acetate solution, and the resulting suspension was stirred at 80°C for 2 hours to perform ion exchange of the H ion-type MFI. That is, H ions of the H ion-type MFI were exchanged with K ions. Subsequently, centrifugation was performed to remove a supernatant. Next, 150 mL of the 0.2 mol/L aqueous potassium acetate solution was added to the solid, followed by stirring. Then, centrifugation was performed to remove a supernatant, and the solid was washed. This washing treatment was repeated, and the washing was performed three times in total. Next, a water washing treatment was repeated twice more. Next, the solid was dried at 100°C for 2 hours, and then baked at 550°C for 5 hours. As a result, K ion-type MFI was obtained.

(Production Example 2)

Cs ion-type MFI was obtained in the same manner as in Production Example 1 except that, unlike in Production Example 1, the 0.2 mol/L aqueous potassium acetate solution was changed to a 0.2 mol/L aqueous cesium acetate solution.

(Example 6)

Decomposition of methyl oleate was performed by the following method using the device 200 illustrated in FIG. 2. An infrared radiation thermometer was used as the thermometer 11. The thermometer 11 was connected to the holder 1, and the temperature of the sample contained in the holder 1 was appropriately measured.

The holder 1 of the device 200 was charged with 300 mg of the K ion-type MFI obtained in Production Example 1, i.e., the zeolite 3 serving as a catalyst, and 500 μL (0.437 g) of methyl oleate serving as a reaction substrate (the organic compound 4), and an argon gas was allowed to flow from the gas supply 6 at 20 mL/min. Subsequently, the temperature was increased to 350°C by irradiation with an electric field of 2.45 GHz of microwaves at 50 W to 80 W using a microwave generator (single-mode resonator, obtained from Ryowa Electronics Co., Ltd.) serving as the irradiator 2. This temperature was maintained for 2,000 seconds to decompose methyl oleate.

A gas, i.e., a product, was cooled to 15°C by a condenser 15, and then taken out from the gas take-out portion 12. Then, quantitative analysis of the components shown in Table 11 below in the gas, i.e., the product, was performed. Using a standard argon gas (obtained from GL Sciences, Inc.) as an external standard, the quantitative analysis was performed in the same manner as in Example 1, i.e., the quantitative analysis of the components was performed through in-line analysis using micro GC with a thermal conductivity detector (TCD) being a detector. The results of the quantitative analysis in Example 6 are shown in Table 11.

(Example 7)

A reaction was performed in the same manner as in Example 6 except that, unlike in Example 6, the K ion-type MFI serving as a catalyst was changed to the Cs ion-type MFI obtained in Production Example 2. Then, quantitative analysis of the components shown in Table 11 was performed in the same manner as in Example 6. The results of the quantitative analysis in Example 7 are shown in Table 11.

[Table 11]

| | | Example 6 | Example 7 |
|---|---|---|---|
| Zeolite | | K ion-type MFI | Cs ion-type MFI |
| Yield [% by mass] | Ethylene | 0.52 | 2.00 |
| | Ethane | 0.03 | 0.31 |
| | Propylene | 2.67 | 4.51 |
| | Propane | 0.10 | 0.33 |
| Total yield [% by mass] | | 3.32 | 7.15 |
| O/P ratio of products having 2 carbon atoms and 3 carbon atoms | | 24.54 | 10.17 |
| Yield [% by mass] of olefins having 2 carbon atoms and 3 carbon atoms | | 3.19 | 6.51 |
| Yield [% by mass] of paraffins having 2 carbon atoms and 3 carbon atoms | | 0.13 | 0.64 |

In the analysis through micro GC, ethylene, ethane, propylene, and propane were steadily produced at 0.65 mL/min, 0.056 mL/min, 1.30 mL/min, and 0.37 mL/min, respectively. Also, a high O/P ratio was obtained when the zeolite ion-exchanged with an alkali metal, such as potassium ions, cerium ions, or the like, was used as a catalyst.

The present international application claims priority to Japanese Patent Application No. 2023-181980, filed on October 23, 2023, and the entire contents of Japanese Patent Application No. 2023-181980 are incorporated in the present international application by reference.

REFERENCE SIGNS LIST

100 : Device

200 : Device

1 : Holder

2 : Irradiator

3 : Zeolite

4 : Organic compound

5 : Organic compound supply

5a : Organic compound flow portion

5b : Introduction portion

6 : Gas supply

6a : Gas flow portion

6b : Pump

7 : Recovery portion

7A : Liquid product recovery portion

7B : Gas recovery portion

7a : Trap

7b : Cooling portion

7c : Organic solvent

7d : Coolant

8 : Reservoir

G : Inert gas

11 : Thermometer

12 : Gas take-out portion

13 : Condenser

## Claims

1. A method for producing a compound, the method comprising:
performing contact decomposition of an organic compound with zeolite irradiated with microwaves, thereby producing at least one selected from the group consisting of a lower olefin and an aromatic compound.

2. A method for producing a compound, the method comprising:
performing decomposition of an organic compound with zeolite irradiated with microwaves, thereby producing at least one selected from the group consisting of a lower olefin and an aromatic compound.

3. The method for producing the compound according to claim 1 or 2, wherein
the organic compound is at least one selected from the group consisting of a fatty acid, a fatty acid ester, and a hydrocarbon.

4. The method for producing the compound according to claim 1 or 2, wherein
the zeolite is at least one selected from the group consisting of an aluminosilicate, a borosilicate, a gallosilicate, and a silicoaluminophosphate.

5. The method for producing the compound according to claim 1 or 2, wherein
the zeolite is at least one selected from the group consisting of ZSM-5 and Y.

6. The method for producing the compound according to claim 1 or 2, wherein
the zeolite is ZSM-5.

7. The method for producing the compound according to claim 1 or 2, wherein
the zeolite contains an alkali metal.

8. The method for producing the compound according to claim 1 or 2, wherein
a temperature of the zeolite is 400°C to 600°C.

9. The method for producing the compound according to claim 1 or 2, wherein
a frequency of the microwaves is 300 MHz to 300 GHz.

10. The method for producing the compound according to claim 1 or 2, wherein
a molecular weight of the organic compound is higher than a molecular weight of the lower olefin and a molecular weight of the aromatic compound.

11. A device for producing a compound, the device comprising:

    a holder configured to hold zeolite; and
    an irradiator configured to irradiate the zeolite with microwaves, wherein
    the device is configured to perform, in the holder, contact decomposition of an organic compound with the zeolite irradiated with the microwaves by the irradiator, thereby producing at least one selected from the group consisting of a lower olefin and an aromatic compound.

12. A device for producing a compound, the device comprising:

    a holder configured to hold zeolite; and
    an irradiator configured to irradiate the zeolite with microwaves, wherein
    the device is configured to perform, in the holder, decomposition of an organic compound with the zeolite irradiated with the microwaves by the irradiator, thereby producing at least one selected from the group consisting of a lower olefin and an aromatic compound.

13. Zeolite for use in performing contact decomposition of an organic compound by irradiation with microwaves, thereby producing at least one selected from the group consisting of a lower olefin and an aromatic compound.

14. Zeolite for use in performing decomposition of an organic compound by irradiation with microwaves, thereby producing at least one selected from the group consisting of a lower olefin and an aromatic compound.

FIG.1

8
100
4
5a
5
5b
6
6a
6b
G
1
2
3
2
7B
7a
7b
7A
7c
7d

# FIG.2

200
12
13
13
G
6b
6
6a
11
2
4
1
3

FIG.3

350
300
250
200
150
100
50
0
TEMPERATURE (°C)
0
50
100
150
200
TIME (sec)
Na ion-type MFI (2.45 GHz)
Na ion-type MFI (5.8 GHz)
H ion-type MFI (2.45 GHz)
H ion-type MFI (5.8 GHz)

**INTERNATIONAL SEARCH REPORT**

International application No.
**PCT/JP2024/023686**

**A. CLASSIFICATION OF SUBJECT MATTER**

***C07C 1/213***(2006.01)i; ***B01J 29/40***(2006.01)i; ***C07B 61/00***(2006.01)i; ***C07C 4/06***(2006.01)i; ***C07C 11/04***(2006.01)i; ***C07C 11/06***(2006.01)i; ***C07C 15/04***(2006.01)i
FI: C07C1/213; C07C4/06; C07C11/04; C07C11/06; C07C15/04; B01J29/40 Z; C07B61/00 D; C07B61/00 300

According to International Patent Classification (IPC) or to both national classification and IPC

**B. FIELDS SEARCHED**

Minimum documentation searched (classification system followed by classification symbols)

C07C1/213; B01J29/40; C07B61/00; C07C4/06; C07C11/04; C07C11/06; C07C15/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | XUE, Z. et al. Microwave-Assisted Catalytic Fast Pyrolysis of Biomass for Hydrocarbon Production with Physically Mixed MCM-41 and ZSM-5. Catalysts. 18 June 2020, vol. 10, no. 6, Articles no. 685, pp. 1-11, doi:10.3390/catal10060685<br>abstract, p. 2, line 10 to p. 3, line 1 from the bottom, p. 4, lines 11-1 from the bottom, p. 6, line 13 from the bottom to p. 7, line 3, p. 7, line 17 from the bottom to p. 8, line 16, p. 9, lines 21-31, fig. 1(a), 2(a), 3, 5(a), 6 | 1-14 |
| Y | | 1-14 |
| Y | LIU, J. et al. Biomass Pyrolysis Technology by Catalytic Fast Pyrolysis, Catalytic Co-Pyrolysis and Microwave-Assisted Pyrolysis: A Review. Catalysts. 04 July 2024, vol. 10, no. 7 Articles no. 742, pp. 1-26, doi:10.3390/catal10070742<br>abstract, p. 2, lines 21-23, p. 4, lines 7-1 from the bottom, p. 14, lines 16-27, p. 15, lines 31-32, p. 15, line 3 from the bottom to p. 16, line 1, tables 2, 3 | 1-14 |

☑ Further documents are listed in the continuation of Box C. ☐ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2024** | **17 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/023686**

**C. DOCUMENTS CONSIDERED TO BE RELEVANT**

| Category* | Citation of document, with indication, where appropriate, of the relevant passages | Relevant to claim No. |
|---|---|---|
| A | KIM, S. et al. Synthesis of ZSM-5 catalysts via microwave-assisted heating method for military jet fuel cracking into petroleum gas. Microporous and Mesoporous Materials. 22 September 2021, vol. 328, Articles no. 111446, pp. 1-25, https://www.sciencedirect.com/science/article/pii/S1387181121005722 fig. 5, table 3 | 1-14 |
| A | NASSER, G. A. et al. Microwave-Assisted Hydrothermal Synthesis of CHA Zeolite for Methanol-to-Olefins Reaction. Industrial & Engineering Chemistry Research. 22 November 2018, vol. 58, no. 1, pp. 60-68, doi:10.1021/acs.iecr.8b04401 tables 4, 5, fig. 12 | 1-14 |

Form PCT/ISA/210 (second sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011526640 W
- JP 2018522086 W
- JP 5833634 B
- JP 5234456 B
- WO 2012121366 A
- JP 2022506131 W
- JP 2023181980 A